(19) Europäisches Patentamt
European Patent Office
Office européen des brevets

(11) **EP 3 174 845 B1**

(12) **EUROPÄISCHE PATENTSCHRIFT**

(45) Veröffentlichungstag und Bekanntmachung des Hinweises auf die Patenterteilung:
**29.04.2020 Patentblatt 2020/18**

(21) Anmeldenummer: **15750272.5**

(22) Anmeldetag: **29.07.2015**

(51) Int Cl.:
**C07C 67/56** (2006.01)

(86) Internationale Anmeldenummer:
**PCT/EP2015/067337**

(87) Internationale Veröffentlichungsnummer:
**WO 2016/016285 (04.02.2016 Gazette 2016/05)**

(54) **VERFAHREN ZUR AUFARBEITUNG EINES ROHESTERS, DER VERESTERUNGSKATALYSATOR-HYDROLYSEPRODUKT IN SUSPENDIERTER PARTIKULÄRER FORM ENTHÄLT**

METHOD FOR THE PROCESSING OF A RAW ESTER CONTAINING THE ESTERIFICATION CATALYST HYDROLYSIS PRODUCT IN SUSPENDED PARTICULATE FORM

PROCÉDÉ DE PRÉPARATION D'UN ESTER BRUT, CONTENANT UN PRODUIT D'HYDROLYSE DE CATALYSEUR D'ESTÉRIFICATION SOUS FORME PARTICULAIRE EN SUSPENSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.08.2014 EP 14179461**

(43) Veröffentlichungstag der Anmeldung:
**07.06.2017 Patentblatt 2017/23**

(73) Patentinhaber: **BASF SE**
**67056 Ludwigshafen am Rhein (DE)**

(72) Erfinder:
• **MAKARCZYK, Piotr**
**67256 Weisenheim am Sand (DE)**
• **BREITSCHEIDEL, Boris**
**67165 Waldsee (DE)**
• **ROESSLER-FEIGEL, Beatrice**
**67256 Weisenheim am Sand (DE)**
• **MUELHEIMS, Kerstin**
**67069 Ludwigshafen (DE)**

(74) Vertreter: **BASF IP Association**
**BASF SE**
**G-FLP-C006**
**67056 Ludwigshafen (DE)**

(56) Entgegenhaltungen:
**WO-A1-2010/076193**

**Beschreibung**

HINTERGRUND DER ERFINDUNG

Die Erfindung betrifft ein Verfahren zur Aufarbeitung eines Rohesters einer Veresterungsreaktion, die mit einem metallhaltigen Veresterungskatalysator katalysiert ist, wobei der Rohester Veresterungskatalysator-Hydrolyseprodukte in suspendierter partikulärer Form enthält.

STAND DER TECHNIK

Ester der Phthalsäure, Adipinsäure, Sebacinsäure oder Maleinsäure finden weite Anwendung in Lackharzen, als Bestandteile von Anstrichmitteln und insbesondere als Weichmacher für Kunststoffe.

Es ist bekannt, Carbonsäureester durch Umsetzung von Carbonsäuren mit Alkoholen herzustellen. Diese Reaktion kann autokatalytisch oder katalytisch, beispielsweise durch Brönstedt- oder Lewissäuren, durchgeführt werden. Vielfach werden Metallverbindungen als Katalysatoren verwendet, wie die Alkoholate, Carboxylate und Chelatverbindungen von Titan, Zirkonium, Zinn, Zink und Aluminium.

Obgleich die katalytischen Eigenschaften dieser metallhaltigen Katalysatoren zufriedenstellend sind, bereitet die Entfernung der Katalysatorrückstände aus den Veresterungsprodukten Schwierigkeiten. Zur Reinigung versetzt man die rohen Ester in der Regel zunächst zur Neutralisation von nicht oder unvollständig umgesetzter Säure (Partialester) mit Alkalihydroxiden und entfernt die freien Alkohole durch Wasserdampfdestillation. Nach kurzer Vakuumdestillation zur Trocknung des Produkts werden dann die Katalysator-Rückstände durch Filtration entfernt. Da die Katalysator-Rückstände in der Regel von schleimiger, gelartiger Konsistenz sind, ist die Filtration meist nur unter Zuhilfenahme von Filtrierhilfsmitteln, wie z. B. Aktivkohle, Holzmehl oder Kieselgur, möglich. Dennoch ist eine derartige Filtration noch mit schwerwiegenden Nachteilen verbunden: Es werden lange Filtrationszeiten benötigt und die Ausbeute an Ester wird verringert, weil im Filterkuchen große Mengen Produkt festgehalten werden.

In der WO 2010/076193 A1 ist ein Verfahren zur Aufarbeitung eines rohen Esters aus einer Veresterungsreaktion, die mit einem metallhaltigen Veresterungskatalysator katalysiert wurde, bekannt. Die Aufarbeitung erfolgt indem man a) den rohen Ester bei einer Temperatur T von mehr als 100 °C unter einem Druck p, der gleich oder größer ist als der Dampfdruck von Wasser bei der Temperatur T, mit einer wässrigen Base versetzt, b) das Ester-Base-Gemisch entspannt und Wasser abdampft, c) die erhaltene flüssige Phase unter Bildung einer Wasser-in-Öl-Emulsion mit Wasser versetzt, d) aus der Emulsion Wasser abdestilliert und e) den Ester filtriert.

Aus der DE 194 53 59 geht ein Verfahren zur Aufarbeitung von rohen Weichmachern hervor, das die folgenden aufeinanderfolgenden Schritte aufweist: (i) die Restsäure im Roh-Weichmacher wird mit alkalischen Stoffen (z. B. 25 %ige Natronlauge) neutralisiert; (ii) die freien Alkohole im Roh-Weichmacher werden durch WasserdampfDestillation entfernt; (iii) das Produkt wird auf Temperaturen abgekühlt, die unter dem Siedepunkt des Wassers beim jeweiligen Druck liegen; (iv) es werden mindestens 0,5 Gewichtsprozent Wasser, bezogen auf das aufzuarbeitende Produkt, zugesetzt; (v) das Gemisch aus Wasser und aufzuarbeitendem Produkt wird mindestens 15 Minuten bei Temperaturen, die unter der Siedetemperatur des Wassers beim jeweiligen Druck liegen, intensiv gerührt; (vi) das zugesetzte Wasser wird durch Vakuumdestillation entfernt; (vii) der Weichmacher wird filtriert. Bei Zugabe der Natronlauge unter den angegebenen Bedingungen verdampft sofort ein wesentlicher Teil des mit der wässrigen Lauge zugeführten Wassers, so dass festes Natriumhydroxid ausfällt. Festes Natriumhydroxid reagiert wesentlich langsamer als gelöstes NaOH. Außerdem führt die Ausfällung zu Ablagerungen an Rohrleitungen und Behältern, die häufiges Reinigen erforderlich machen.

Die DE 23 30 435 beschreibt ein Verfahren zur Aufarbeitung von rohen Estern, bei dem man den 140 bis 250 °C heißen rohen Ester bei vermindertem Druck gleichzeitig mit wässrigen Lösungen von Alkali- oder Erdalkalihydroxid neutralisiert und durch Versetzen mit Wasser bei vermindertem Druck einer Wasserdampfdestillation unterwirft, anschließend trocknet und die gebildeten festen Bestandteile abfiltriert. Der Druck und die Geschwindigkeit der Wasserzugabe sollen so geregelt werden, dass das zugegebene Wasser rasch verdampft.

Unter den Verfahrensbedingungen, bei denen zugegebenes Wasser sofort verdampft, kann festes Alkali- oder Erdalkalihydroxid ausfallen, was zu den vorstehend beschriebenen Nachteilen führt. Da festes Hydroxid wesentlich langsamer reagiert, sind zur vollständigen Neutralisation bisweilen hohe Basenüberschüsse erforderlich.

Die EP 1 300 388 offenbart ein Verfahren zur Herstellung von Carbonsäureestern, wobei der überschüssige Alkohol nach der Veresterungsreaktion entfernt, der so erhaltene Rohester durch Basenzugabe neutralisiert und anschließend filtriert wird. Der Alkohol wird durch mindestens eine Wasserdampfdestillation abgetrennt und die Basenzugabe erfolgt während einer Wasserdampfdestillation. Die Lauge soll unten in das Reaktionsgemisch eingedüst werden. Durch die hohe Temperatur verdampft das Wasser. Durch geringe Zudosierungsgeschwindigkeiten der Lauge sollen Nebenreaktionen, wie beispielsweise die Verseifung der Ester, gering gehalten werden. Dies hat aber den Nachteil langer Neutralisationszeiten bzw. geringer Durchsätze.

Die US 5,434,294 beschreibt ein Verfahren zur Titanat-katalysierten Herstellung von Weichmacherestern. Das Produkt wird mit wässriger Base behandelt und dann unter Zuhilfenahme eines Filtrierhilfsmittels wie Bleicherde, Hydrotalcit oder Magnesiumsilikat, filtriert. Das Vorlegen eines Wasser-untersättigten Rohesters, wodurch die suspendierten partikulären Veresterungskatalysator-Hydrolyseprodukte stabile Agglomerate ausbilden, ist nicht bekannt.

Die WO 97/11048 veranschaulicht die Herstellung von gemischten Phthalsäureestern. Die Umsetzung eines Phthalsäurehalbesters mit einem Polyethylenglykolmonomethylether wird mit Tetraisopropyl-Titan katalysiert. Nach beendeter Umsetzung wird tropfenweise Natriumbicarbonatlösung zugegeben. Nach dem Abkühlen gibt man 2 % Wasser zu, destilliert flüchtige Verbindungen, wie Wasser und Lösungsmittel, im Vakuum ab und filtriert.

Die DE 197 21 347 offenbart ein Verfahren zur Herstellung von Esterweichmachern, bei dem man eine Mischung von Säure oder Säureanhydrid und Alkohol zunächst bei 100 bis 160 °C unter Entfernung gegebenenfalls gebildeten Wassers miteinander reagieren lässt, die Reaktion unter Zusatz des Katalysators und durch Erhöhen der Temperatur bis auf 250 °C zu Ende führt, das Reaktionsgemisch mit einer wässrigen Alkali- oder Erdalkalihydroxid-Lösung umsetzt, darauf den überschüssigen Alkohol abtrennt, den zurückbleibenden Rohester trocknet und filtriert. Die alkalische Behandlung soll zweckmäßigerweise unmittelbar im Anschluss an den Veresterungsschritt ohne vorherige Abkühlung des Reaktionsgemisches erfolgen.

Die US 2006/0270868 A1 beschreibt ein Verfahren zur Reinigung eines Rohesters einer Veresterungsreaktion, die mit einem Veresterungskatalysator katalysiert wurde und einer Behandlung mit einer basischen, wässrigen Alkalimetallsalzlösung bei einem Wassergehalt im Bereich von 0,7 bis 1,4 Gew.-% bezogen auf das Gewicht des Rohesters. Vorzugsweise wird ein Titankatalysator eingesetzt, was zur Verbesserung der Filtrierbarkeit und der Reinheit der hergestellten Esters führt.

Bekannte Verfahren zur Aufarbeitung eines suspendiert partikulär Veresterungskatalysator-Hydrolyseprodukt enthaltenden Rohesters sind noch verbesserungswürdig und haben oft den Nachteil der schlechten Abtrennbarkeit der Veresterungskatalysatorreste, von langen Verfahrenszeiten, von Verstopfungen und Ausfällen von Filtereinheiten und schlecht regelbaren Verfahrensparametern.

Der vorliegenden Erfindung liegt die Aufgabe zugrunde, ein Verfahren zur Aufarbeitung eines rohen Estergemisches anzugeben, das mit hohem Durchsatz in gut reproduzierbarer Weise zu Estern mit niedriger Säurezahl führt und bei dem die festen Katalysator-Rückstände in kurzer Zeit agglomerieren und in gut abfiltrierbarer Form anfallen.

ZUSAMMENFASSUNG DER ERFINDUNG

Die Aufgabe wird überraschenderweise gelöst durch ein Verfahren zur Aufarbeitung eines Rohesters, der Veresterungskatalysator-Hydrolyseprodukte in suspendierter partikulärer Form enthält, bei dem man

a) den Rohester in einem Emulgier-Kessel mit 1 bis 10 Gew.-% Wasser versetzt und das Wasser unter Erhalt einer Suspo-Emulsion im Rohester emulgiert,
b) die Suspo-Emulsion aus dem Emulgier-Kessel in einen Agglomerier-Kessel mit vorgelegtem, Wasser-untersättigten Rohester überführt, so dass sich im Gemisch der Suspo-Emulsion und des vorgelegten Rohesters ein Wassergehalt unterhalb der Löslichkeitsgrenze von Wasser im Rohester einstellt, wodurch die suspendierten partikulären Veresterungskatalysator-Hydrolyseprodukte stabile Agglomerate ausbilden, und
c) die gebildeten Agglomerate abfiltriert.

Das erfindungsgemäße Verfahren umfasst mehrere Schritte: eine Emulgation (Schritt a)), eine Agglomeration (Schritt b)) und eine Filtration (Schritt c)).

Das Verfahren kann kontinuierlich durchgeführt werden, wobei die einzelnen Schritte in hintereinander geschalteten kontinuierlich betriebenen Apparaturen durchgeführt werden. Alternativ kann das Verfahren diskontinuierlich durchgeführt werden.

DETAILLIERTE BESCHREIBUNG DER ERFINDUNG

Das erfindungsgemäße Verfahren geht von einem Rohester aus, der Veresterungskatalysator-Hydrolyseprodukte in suspendierter partikulärer Form enthält. Dieser Rohester wird im Allgemeinen erhalten, indem man das Veresterungsprodukt einer Veresterungsreaktion, die mit einem metallhaltigen Veresterungskatalysator katalysiert ist, mit einer wässrigen Base versetzt und anschließend Wasser aus dem Veresterungsprodukt-Base-Gemisch abdampft Durch Zugabe einer wässrigen Base wird der zur Synthese eingesetzte Veresterungskatalysator durch Hydrolyse desaktiviert und ausgefällt. Gleichzeitig werden die bei der Veresterungsreaktion nicht umgesetzte Säure bzw. Partialester der Säure in Salze überführt. Die zugegebene Menge wässrige Base ist so bemessen, dass sie zur vollständigen Neutralisation der sauren Komponenten des rohen Esters ausreicht. In der Praxis wird ein mehr oder weniger großer Überschuss an Base eingesetzt. Die Gesamtmenge der sauren Komponenten des rohen Esters wird zweckmäßig durch die Säurezahl SZ in mg KOH/g erfasst.

In einer bevorzugten Ausführungsform weist der Rohester vor der Neutralisation eine Säurezahl von kleiner als 0,1 mg KOH/g Rohester auf. Es wurde gefunden, dass bei höherer Säurezahl eine kontinuierliche Filtration der gebildeten Agglomerate nur sehr schwer möglich bis unmöglich ist, da das Filtermedium zusetzt und verstopft. Es wird angenommen, dass höhere Säurezahlen nach der Neutralisation die Anwesenheit höherer Konzentrationen von Partialester-Salzen bedingen, die als Emulgatoren wirken. Vermutlich reichern sich die Partialester-Salze in der wässrigen Phase an und erschweren die Ausbildung definierter Agglomerate. Des Weiteren können die nach der Entfernung der sichtbaren Wasserphase in den Agglomeraten verbleibenden Partialester-Salze die Eigenschaften der Agglomerate verändern, so dass diese nicht mehr zufriedenstellend filtrierbar sind.

Vorzugsweise bringt man mit der wässrigen Base 100 bis 300 % Neutralisationsäquivalente ein, bezogen auf die Säurezahl des rohen Esters, insbesondere 130 bis 220 %. Unter Neutralisationsäquivalent wird dabei der fiktive Bruchteil eines Base-Moleküls verstanden, der ein Proton binden kann. Mit anderen Worten verwendet man einen Basenüberschuss von bis zu 200 %, vorzugsweise im Bereich von 30 bis 120 %.

Als wässrige Base kommen Lösungen von Hydroxiden, Carbonaten, Hydrogencarbonaten von Alkalimetallen und Erdalkalimetallen in Betracht. Wässrige Alkalimetallhydroxidlösungen sind im Allgemeinen bevorzugt. Wässrige Natriumhydroxidlösung ist aufgrund ihrer leichten Verfügbarkeit besonders bevorzugt.

Die Konzentration der wässrigen Base ist an sich nicht kritisch, jedoch kann es beim Einsatz konzentrierter Alkalilösungen an der Einleitstelle der Base zur Hydrolyse der Ester kommen. Andererseits soll die Konzentration der wässrigen Base nicht zu niedrig sein, da das mit der wässrigen Base eingebrachte Wasser im nachfolgenden Schritt wieder entfernt werden muss. Daher sind wässrige Basen mäßiger bis geringer Konzentration bevorzugt, z. B. solche mit einer Konzentration von 0,5 bis 25 Gew.-%, insbesondere von 1 bis 10 Gew.-%. wässrige Natriumhydroxidlösung mit einer Konzentration von 1 bis 5 Gew.-% ist besonders bevorzugt.

Vorzugsweise versetzt man das rohe Veresterungsprodukt bei einer Temperatur T von mehr als 100 °C unter einem Druck p, der gleich oder größer ist als der Dampfdruck von Wasser bei der Temperatur T, mit der wässrigen Base. Es wurde festgestellt, dass eine ausreichend schnelle und vollständige Neutralisation erreicht wird, wenn die wässrige Base bei einer Temperatur T von mehr als 100 °C unter einem Druck p, der gleich oder größer ist als der Dampfdruck von Wasser bei der Temperatur T, zugegeben wird. Das rohe Veresterungsprodukt, der nach der Veresterungsreaktion bzw. nach der Abtrennung von überschüssigem Alkohol vorliegt, weist im Allgemeinen eine erhöhte Temperatur auf. Es kann gegebenenfalls abgekühlt werden, jedoch nur soweit, dass seine Temperatur noch mehr als 100 °C beträgt. Die Zugabe der wässrigen Base erfolgt unter Druckbedingungen, unter denen das Wasser nicht spontan verdampft. Die Base steht daher für die Neutralisationsreaktion vollständig in gelöster, flüssiger Form zur Verfügung. Dies beschleunigt die Reaktion und erlaubt einen vollständigen Umsatz. Würde die wässrige Base bei geringerem Druck zugegeben werden, würde Wasser verdampfen und die gelöste Base als Feststoff ausfallen. Die feste Base stünde nicht oder nur mit deutlich geringerer Reaktionsgeschwindigkeit für die Neutralisation zur Verfügung.

In der Regel weist das rohe Veresterungsprodukt eine Temperatur T von 120 bis 185 °C auf. Der zugehörige Dampfdruck $p_{vap}$ von Wasser kann der nachstehenden Tabelle oder dem Fachmann bekannten Nachschlagewerken entnommen werden. Dem Fachmann ist bekannt, dass der Dampfdruck von Lösungsmitteln von gelösten Stoffen oder Mischungsphänomenen beeinflusst wird. Diese Einflüsse können vorliegend vernachlässigt werden. Für die Zwecke der vorliegenden Erfindung wird auf den Dampfdruck von reinem Wasser abgestellt.

Tabelle: Dampfdruck von Wasser

| T [°C] | $p_{vap}$ [bar] |
|--------|-----------------|
| 105 | 1.208 |
| 110 | 1.432 |
| 115 | 1.690 |
| 120 | 1.985 |
| 125 | 2.320 |
| 130 | 2.700 |
| 135 | 3.128 |
| 140 | 3.613 |
| 145 | 4.154 |
| 150 | 4.758 |

(fortgesetzt)

| T [°C] | $p_{vap}$ [bar] |
|---|---|
| 160 | 6.179 |
| 170 | 7.917 |
| 180 | 10.026 |
| 190 | 12.549 |
| 200 | 15.547 |

In der Regel ist der Druck p, bei dem die wässrige Base zugegeben wird, höher als der Dampfdruck $p_{vap}$ bei der Temperatur T. Vorzugsweise beträgt der Druck p wenigstens das 1,1-fache von $p_{vap}$, insbesondere wenigstens das 1,25-fache von $p_{vap}$. Drücke von mehr als 25 bar sind technisch nur aufwendig zu realisieren und daher nicht bevorzugt.

Die Zugabe der wässrigen Base kann auf beliebige geeignete Weise erfolgen. Sie erfolgt vorzugsweise unterhalb der Flüssigkeitsoberfläche des rohen Veresterungsprodukts. Hierfür eignen sich z. B. Lanzen oder Düsen, die an einem Behälterboden oder der Behälterwand vorgesehen sind. Das Gemisch wird dann intensiv durchmischt, z. B. mittels Rührer oder einer Umwälzpumpe.

Bei kontinuierlicher Durchführung führt man die Basenzugabe zweckmäßigerweise durch, indem man die wässrige Base in einen Strom des rohen Veresterungsprodukts einspritzt. Zur homogenen Einmischung der wässrigen Base führt man den gemischten Strom durch wenigstens einen Mischer. Hierbei kommen dynamische Mischer oder statische Mischer oder Kombinationen davon in Betracht. Statische Mischer sind bevorzugt. Die statischen Mischer lassen sich strömungsmechanisch in Turbulenz- und Laminarmischer unterteilen. Bei den Turbulenzmischern kommen sowohl freie turbulenzerzeugende Mischsysteme als auch solche mit Einbauten in Betracht. Zu den geeigneten statischen Mischern zählen Multiflux-Mischer, Wendelmischer, Wirbelmischer, Gittermischer, Sulzer-SMX-Mischer, Sulzer-SMV-Mischer und Kenics-Mischer. In einer geeigneten Ausführungsform ist der statische Mischer ein Rohr mit einer querschnittsverengenden Blende. Der Drucksprung hinter der Blende erzeugt eine Turbulenz, die zu einer ausreichenden Vermischung führt.

Nach Zugabe der Base wird das mit der wässrigen Base eingebrachte Wasser entfernt. Hierzu wird das mit der wässrigen Base versetzte rohe Veresterungsprodukt zweckmäßigerweise entspannt, z. B. auf einen Druck von weniger als 800 mbar, insbesondere weniger als 250 mbar, z. B. im Bereich von 50 bis 150 mbar. Auf diese Weise kann das mit der wässrigen Base eingebrachte Wasser entfernt werden, ohne den Rohester übermäßig thermisch zu belasten. Infolge der Entspannung trennt sich das Gemisch in eine flüssige Phase und eine Dampfphase. Mit der Dampfphase, die abgezogen wird, wird das mit der wässrigen Base eingeführte Wasser wieder entfernt. Neben dem mit der wässrigen Base eingeführten Wasser dampft bei dieser Behandlung gewöhnlich auch ein Teil des Restalkohols ab. Die Wasser und Alkohol enthaltenden Brüden können gesammelt und kondensiert werden und verworfen oder einer Wiederverwendung zugeführt werden.

Die flüssige Phase weist nach der Entspannung im Allgemeinen eine Temperatur von 130 bis 200 °C auf. Hierzu kann bei Bedarf die flüssige Phase erwärmt werden.

Die Art des Entspannungsgefäßes ist nicht kritisch. Z. B. kann man das Gemisch in einen Rührkessel entspannen, in dem eine weitere Behandlung der flüssigen Phase erfolgt.

Zur Vervollständigung des Abdampfens des Wassers ist es bevorzugt, die bei der Entspannung erhaltene flüssige Phase während einer Verweilzeit von z. B. 5 Minuten bis 1 Stunde, insbesondere 10 bis 40 Minuten, unter vermindertem Druck mechanisch zu bewegen. Hierzu eignen sich Rührer verschiedener Bauarten, wie z. B. ein Kreuzbalkenrührer.

Das im Rohester enthaltene suspendiert partikuläre Veresterungskatalysator-Hydrolyseprodukt als ausgefallener Feststoff umfasst im Wesentlichen Katalysator-Zersetzungsprodukte und Salze von nicht umgesetzter Säure bzw. Partialestern mehrbasiger Säuren, in fein verteilter, schwer filtrierbarer Form. Das erfindungsgemäße Verfahren sieht daher Maßnahmen vor, bei denen die feinen Teilchen zu größeren, leicht abtrennbaren Teilchen agglomeriert werden.

Hierzu versetzt man in Schritt a) die flüssige Phase in einem Emulgier-Kessel mit Wasser unter Bildung einer Suspo-Emulsion. Das Wasser wird als disperse Phase in Form feiner Tröpfchen in der flüssigen organischen Phase verteilt. Es wird angenommen, dass sich die feinen Feststoffteilchen an der Grenzfläche zwischen Wassertröpfchen und umgebender organischer Phase und/oder in den Wassertröpfchen sammeln. Das sich bildende ternäre Gemisch wird im Rahmen der vorliegenden Erfindung als Suspo-Emulsion bezeichnet; hierunter wird eine Emulsion verstanden, die eine disperse wässrige Suspension in einer kontinuierlichen Öl-Phase umfasst.

Damit sich eine eigene Wasserphase ausbildet, muss die zugegebene Wassermenge größer sein, als der Löslichkeit von Wasser in der organischen Phase entspricht. Die Wasserlöslichkeit in der organischen Phase hängt unter anderem vom Gehalt an nicht umgesetzten Alkohol ab, da der Alkohol als Lösungsvermittler wirkt. Je höher der Alkoholgehalt, umso mehr Wasser muss im Schritt a) zugesetzt werden. Bei üblichen Restalkoholgehalten von 1 bis 3 Gew.-% sind im allgemeinen Mengen von 1 bis 10 Gew.-% Wasser, vorzugsweise 2 bis 6 Gew.-% Wasser, bezogen auf das Gewicht des Rohesters, geeignet.

Der Emulgiervorgang kann mit einem geeigneten Rührer, Rührer-Stator-System oder Homogenisator in einem Emulgier-Kessel durchgeführt werden. Die flüssige Wasserphase wird hierbei in feine Wassertropfen zerteilt. Geeignete Rührer, die auch als Rührorgan bezeichnet werden, sind ausgewählt unter Radialrührern, insbesondere Kreuzbalkenrührern, Gitterrühreren, Blattrührern, Ankerrührern, Wendelrührern, MIG-Rührern, Scheibenrührern, Schrägblattrührern, Propellerrührern, Impellerrührern, Turbinenrührern, schneckenförmigen Rührern, Halbmondrührern und Kombinationen hiervon. Als Rührer mit hoher spezifischer Rührleistung eignen sich beispielsweise Scheibenrührer. Alternativ kann man besonders bei kontinuierlicher Verfahrensführung eine Mischdüse verwenden, bei der über ein Dispergierventil Wasser direkt in den Rohesterstrom zugegeben wird. In einer Ausführungsform kann zum Durchmischen auch ein Behälter mit einem Umpumpkreislauf mit einer Umwälzpumpe vorgesehen sein.

Es wurde gefunden, dass für eine optimale Emulgierung der volumenspezifische Leistungseintrag und/oder die Umfangsgeschwindigkeit kritisch sind. In einer bevorzugten Ausführungsform emulgiert man in Schritt a) das zugegebene Wasser mit mindestens einem Mischer bei einer Reynoldszahl von mehr als $10^4$ bei einem volumenspezifischen Leistungseintrag im Bereich von 0,25 bis 2,2 kW/m$^3$, vorzugsweise 0,6 bis 2,0 kW/m$^3$, im Rohester. Niedrigere als die angegebenen Leistungseinträge führen zu groben instabilen Suspo-Emulsionen, die rasch koagulieren. Höhere als die angegebenen Leistungseinträge führen zu hochdispersen Suspo-Emulsionen, die im Schritt b) sehr kleinteilige Agglomerate bilden, welche schwer filtrierbar sind, oder bei denen keine Agglomerate ausgebildet werden, da die Tröpfchengröße der Suspo-Emulsion kleiner als oder vergleichbar der Primärteilchengröße der Veresterungskatalysator-Hydrolyseprodukte ist.

Der Leistungseintrag eines Mischers in ein mehrphasiges System ist eine dem Fachmann bekannte Größe. Im Rahmen der vorliegenden Erfindung wird der Leistungseintrag für exemplarische Rotor-Stator Systeme sowie für Rührer für wie nachfolgend beschrieben experimentell bestimmt.

Für ein Rotor-Stator-Rührsystem wird in einem adiabaten Rührgefäß eine Anfangsmenge eines zu rührenden Systems mit einer oder mehreren Phase(n) und einer Dichte vorgelegt und das Rotor-Stator-Rührsystem in dem Rührgefäß angeordnet. Die Anfangstemperatur des zu rührenden Systems wird gemessen, das Rotor-Stator-Rührsystem gestartet und die Temperaturänderung mit dem Einbringen von Scherkräften in das zu rührenden System durch das Rotor-Stator-Rührsystem über eine Rührzeit t bei einer Drehzahl n des Rotor-Stator-Rührsystems gemessen. Zudem wird die Endtemperatur des zu rührenden Systems gemessen. Mit den ermittelten Daten wird der spezifische Leistungseintrag in das zu rührende System berechnet. Die nachfolgenden Berechnungen werden am Beispiel von Wasser als zu rührendes System durchgeführt.

Der Leistungseintrag wird nach Formel 1 berechnet:

$$\text{Formel 1:} \quad P = [(mW_{quer} * cpW_{quer} * \Delta T) + (\Delta mW * \Delta H_{quer})]/ \, t,$$

wobei P der Leistungseintrag in Watt, $mW_{quer}$ die mittlere Wassermenge in kg, $cpW_{quer}$ die mittlere Wärmekapazität von Wasser in kJ/(kg*K), $\Delta T$ die Differenz zwischen Anfangs- und Endtemperatur des Wassers in °C, $\Delta mW$ die Differenz zwischen Anfangs- und Endwassermenge in kg, $\Delta H_{quer}$ die mittlere Verdampfungsenthalpie von Wasser in kJ/kg und t die Rührzeit in s ist.

Für Systeme mit Rührorganen (Rührer-Systemen) wird bei einer gegebenen Drehzahl n in min$^{-1}$ im Reaktionsprodukt das Drehmoment M in N*cm gemessen.

Die Leistung berechnet sich mit nachfolgender Formel 2 wie folgt:

$$\text{Formel 2:} \quad P = M * 2 * pi * n,$$

wobei M das Drehmoment in N*m und n die Drehzahl in s$^{-1}$ ist.

Alternativ kann die Leistung eines Rührers über die Stromaufnahme des Rührerantriebs ermittelt werden, wobei die auf diese Weise ermittelte Leistung die Antriebsenergieverluste und/oder die Reibungsverluste, insbesondere durch Reibung in Lagern und Dichtungen beinhaltet.

Mit dem in der Formel 1 und oder Formel 2 bestimmten Leistungseintrag P wird die dimensionslose Newton-Leistungskennzahl Ne berechnet nach Formel 3:

Formel 3:    $Ne = P / (\rho W * n^3 * d_2^5)$,

wobei P der volumenspezifische Leistungseintrag in Watt, pW die Dichte von Wasser in $kg/m^3$, n die Rührerdrehzahl in $s^{-1}$ und $d_2$ der Rührorgandurchmesser in m ist.

In einem turbulenten Strömungsbereich in Rührersystemen ist die Ne-Zahl nahezu konstant. Die dimensionslose Reynolds-Zahl wird mit nachfolgender Formel 4 ermittelt.

Formel 4:   $Re = n * d_2^2 * (\rho/\eta)$,

wobei n die Rührerdrehzahl in $s^{-1}$, $d_2$ der Rührorgandurchmesser in m, $\rho$ die Dichte des zu rührenden Systems in $kg/m^3$ und $\eta$ die dynamische Viskosität des zu rührenden Systems in Pa*s ist.

Für Strömungssysteme in welchen Rührer eingesetzt werden, kann eine turbulente Strömung in dem Bereich von Reynoldszahlen von größer als 1000 angenommen werden.

Mit nachfolgender Formel 5 wird der mit der Formel 3 für Wasser bestimmte Newtonzahl auf ein spezifisches zu rührenden Systems mit einer spezifischen Dichte übertragen, wobei das Volumen des Systems berücksichtigt und der volumenspezifische Leistungseintrag Ps bestimmt wird:

Formel 5:   $Ps = Ne * (\rho s * n^3 * d_2^5) / V$,

wobei Ps der volumenspezifische Leistungseintrag in $Watt/m^3$, Ne die dimensionslose Newton-Leistungskennzahl, ps die spezifische Dichte des spezifischen zu rührenden Systems in $kg/m^3$ und $d_2$ der Rührorgandurchmesser in m und V das Volumen in $m^3$ der zu rührenden Flüssigkeit ist.

Der volumenspezifische Leistungseintrag Ps in Watt wird berechnet mit Bezug auf das Volumen des zu rührenden Systems.

In einer bevorzugten Ausführungsform emulgiert man in Schritt a) das zugegebene Wasser mit mindestens einem Rührer bei einer Umfangsgeschwindigkeit im Bereich von 2,8 bis 6,2 m/s, vorzugsweise im Bereich von 3,3 bis 5,6 m/s, besonders bevorzugt im Bereich von 3,8 bis 5,1 m/s im Rohester.

In einer bevorzugten Ausführungsform weist der Emulgier-Kessel eine zylindrische Grundform auf, welche mit einem Boden und vorzugsweise mit einem Deckel abgeschlossen ist. Die Boden- und Deckelformen sind ausgewählt unter Klöpperböden, halbelliptische Böden, Korbbogenböden, ebenen Böden, konischen Böden, Formen für Böden und Deckel nach dem ASME-Standard (American Society of Mechanical Engineering) und Kombinationen hiervon.

In einer bevorzugten Ausführungsform weist der Emulgier-Kessel einen Schlankheitsgrad H/D in einem Bereich von 1 bis 6, vorzugsweise im Bereich von 2 bis 4 auf. Im Rahmen der vorliegenden Erfindung wird unter Schlankheitsgrad H/D eines Behälters, das Verhältnis zwischen der Behältergesamthöhe H (einschließlich des Bodens und des Deckels) und dem Behälterdurchmesser D verstanden.

In einer bevorzugten Ausführungsform weist der Emulgier-Kessel an der Behälterinnenwandung angeordnete Strombrecher, insbesondere in einer Anzahl von 3 bis 12, vorzugsweise von 4 bis 8, je Druck- und Saugseite, auf.

Im Rahmen der vorliegenden Erfindung werden unter Strombrechern, auch bekannt als Stromstörer, strömungsbrechende Einbauten in Rührkesseln verstanden. Strombrecher verhindern ein Mitrotieren des durch ein Rührwerk bewegten Mediums in dem Rührkessel. Strombrecher sind beispielsweise an der Behälterinnenwand oder am Boden innerhalb des Rührkessels installiert.

In einer bevorzugten Ausführungsform weist der Emulgier-Kessel an der Behälterinnenwandung angeordnete Strombrecher mit einem Verhältnis von Strombrecherbreite zu Behälterdurchmesser im Bereich von 0,05 bis 0,5, vorzugsweise im Bereich von 0,1 bis 0,35 auf. Im Rahmen der vorliegenden Erfindung wird unter Strombrecherbreite die Breite des Strombrechers in die von der Behälterinnenwandung in den Behälter hineinragende Richtung verstanden.

In einer bevorzugten Ausführungsform weist der Emulgier-Kessel an der Behälterinnenwandung angeordnete Strombrecher mit einer Strombrecherlänge im Bereich zwischen dem halben Emulgier-Kesseldurchmesser bis maximal zu der Höhe des Emulgier-Kessels auf.

In einer bevorzugten Ausführungsform weist der Emulgier-Kessel einen Füllgrad definiert durch das Verhältnis des Flüssigkeitsfüllstandes $H_f$ bezogen auf die Behältergesamthöhe H in einem Bereich von 0,5 bis 0,9, vorzugsweise in einem Bereich von 0,6 bis 0,8 auf.

Die Wassertröpfchen der so erhaltenen Suspo-Emulsion weisen im Allgemeinen eine mittlere Teilchengröße von mehr als 10 bis weniger als 2000 $\mu$m, insbesondere 200 bis 1000 $\mu$m, auf, bestimmbar z.B. durch Laserbeugungsmethoden.

Der Schritt a) erfolgt zweckmäßigerweise bei etwa Normaldruck.

Für den Schritt b) wird in einem Agglomerier-Kessel Wasser-untersättigter Rohester vorgelegt. "Wasser-untersättigt" bedeutet, dass der Rohester einen Wassergehalt aufweist, der geringer ist als die Löslichkeit von Wasser im Rohester unter den Bedingungen im Agglomerier-Kessel. Der Wasser-untersättigte Rohester vermag Wasser einzulösen, ohne dass sich eine diskrete Wasserphase ausbildet. Der vorgelegte Wasser-untersättigte Rohester kann, insbesondere bei kontinuierlicher oder semikontinuierlicher Verfahrensführung, Agglomerate von Veresterungskatalysator-Hydrolyseprodukten enthalten.

Die Suspo-Emulsion aus dem Emulgier-Kessel wird dann in den Agglomerier-Kessel mit dem vorgelegten, Wasser-untersättigten Rohester überführt, z. B. umgepumpt. Die relativen Mengen von vorgelegtem Rohester und Suspo-Emulsion und der Grad der Wasser-Untersättigung des vorgelegten Esters werden so gewählt, dass sich im Gemisch der Suspo-Emulsion und des vorgelegten Rohesters ein Wassergehalt unterhalb der Löslichkeitsgrenze von Wasser im Rohester einstellt. Durch das Unterschreiten der Löslichkeitsgrenze von Wasser im Gemisch kann das Wasser nicht mehr als eigene Phase existieren. Den Wassertröpfchen wird schlagartig Wasser entzogen und die in den Wassertröpfchen suspendierten bzw. um die Wassertröpfchen angelagerten partikulären Veresterungskatalysator-Hydrolyseprodukte ballen sich zusammen und bilden stabile Agglomerate aus, die grobe, gut abtrennbare Teilchen bilden.

Die Löslichkeit von Wasser im Rohester hängt von verschiedenen Faktoren ab, insbesondere aber von dem Gehalt an nicht umgesetzten Alkohol und der Temperatur. Alkohol kann in Abhängigkeit von der Konzentration als Lösungsvermittler wirken. Die Löslichkeit von Wasser ist abhängig von dem zugehörigen Alkoholgehalt. Durch Zusatz von (nicht verestertem) Alkohol kann daher eine gewünschte Löslichkeit von Wasser im Rohester eingestellt werden. Höhere Temperaturen begünstigen die Löslichkeit von Wasser ebenfalls. Die Temperatur des vorgelegten Rohesters beträgt daher vorzugsweise 60 bis 80 °C, insbesondere 65 bis 75 °C.

Das Überführen der Suspo-Emulsion in die Agglomerate enthaltende Suspension muss einstufig und schnell erfolgen. Um bei einer kontinuierlichen oder semi-kontinuierlichen Verfahrensführung die Wasser-Unsättigung des im Agglomerier-Kessels vorgelegten Rohesters aufrecht zu erhalten, muss aus dem Agglomerier-Kessel kontinuierlich das gelöste Wasser entzogen werden, vorzugsweise indem man kontinuierlich Wasser abdestilliert. Vorzugsweise wird ein Blasensieden vermieden. Hierzu kann die Suspension durch einen Verdampfer, z. B. einen Fallfilmverdampfer, geführt werden. Alternativ kann man die Suspension unter vermindertem Druck mechanisch bewegen, z. B. rühren. Das Rühren kann im Agglomerier-Kessel erfolgen, zweckmäßigerweise unter wenig scherenden Bedingungen. Ein übermäßiger Eintrag von Scherenergie könnte die noch labilen Agglomerate der festen Katalysatorrückstände wieder zu unerwünschten feinteiligen Partikeln zerteilen. In einer bevorzugten Ausführungsform mischt man im Agglomerier-Kessel die Suspo-Emulsion mit mindestens einem Mischer bei einem volumenspezifischen Leistungseintrag von weniger als 0,2 kW/m$^3$ ein.

Vorzugsweise destilliert man das Wasser bei einer Temperatur von 60 bis weniger als 100 °C und einem Druck von weniger als 500 mbar, ab. Gewünschtenfalls kann man das gelöste Wasser auch in mehreren Schritten in aufeinander folgenden Rührbehältern abdestillieren, wobei im zweiten oder weiteren Schritt ein niedrigerer Druck und/oder eine höhere Temperatur als im vorhergehenden Schritt angewandt wird. Das Überführen von einem Rührbehälter in den darauffolgenden Rührbehälter erfolgt vorzugsweise unter wenig scherenden Bedingungen, z. B. durch freien Überlauf und nicht durch Umpumpen. Bei mehrstufiger Durchführung der Entfernung des gelösten Wassers kann es empfehlenswert sein, Rohester aus der letzten Stufe (d.h. Rohester mit dem geringsten Wassergehalt) in den Agglomerier-Kessel zurückzuführen, um im Agglomerierkessel die Wasser-Unsättigung des vorgelegten Rohesters zu begünstigen.

Neben dem in der Suspo-Emulsion enthaltenem Wasser destilliert bei dieser Behandlung gewöhnlich auch ein Teil des Restalkohols ab. Die Wasser und Alkohol enthaltenden Brüden können gesammelt und kondensiert werden und verworfen oder einer Wiederverwendung zugeführt werden.

Nach dieser Behandlung liegt der Feststoff in gut filtrierbarer Form vor; es schlägt kein Feinanteil bei der Filtration in Schritt c) durch. Die Anwendung von Filtrierhilfsmitteln ist nicht erforderlich; ihre Verwendung ist nicht bevorzugt.

Aus dem Agglomerier-Kessel kann Rohester mit darin suspendierten Agglomeraten abgezogen und einer Filtrationseinheit zugeführt werden. Zur Filtration des Esters eignen sich alle geeigneten Filter wie Kammerfilterpressen, Bandfilter, Kerzenfilter oder Tellerfilter. Zur kontinuierlichen Verfahrensführung eignen sich besonders Tellerfilter mit Zentrifugalkuchenabwurf. Der abgetrennte Feststoff wird im Allgemeinen verworfen.

Vorzugsweise erfolgt die Filtration der Agglomerate enthaltenden Suspension in Abwesenheit vom freien Wasser. Die Agglomerate sind hydrophil; beim Kontakt mit Wasser zerfallen sie erneut in nicht filtrierbare nanopartikuläre Feststoffe. Freies Wasser kann beispielweise entstehen, wenn die Suspension gelöstes Wasser enthält. Durch Herabsetzten der Temperatur im Filtrationsschritt, z.B. im Kontakt mit kalten Filtriervorrichtungen, wird die Wasserlöslichkeit im Rohester erniedrigt und es entsteht freies Wasser. Bei der Verwendung von semi-kontinuierlichen Filtern müssen diese nach der Reinigung mit Wasser oder Dampf gründlich getrocknet werden, damit kein freies Wasser in den Filtrationsprozess gelangt.

Der Feststoff kann unter einer hohen relativen Luftfeuchtigkeit Wasser absorbieren, wodurch sich die Agglomerate zersetzen und in eine wässrige Paste überfuhrt werden die nur sehr schlecht oder gar nicht filtrierbar ist. In einer bevorzugten Ausführungsform führt man den Schritt c) daher unter Ausschluss von Feuchtigkeit, insbesondere in einer Umgebung mit trockener Luft und/oder Inertgas.

In einer bevorzugten Ausführungsform unterzieht man das Filtrat aus Schritt c) als flüssige Phase der Suspension einer Strippbehandlung, ausgewählt unter einer Durchströmung mit Dampf, insbesondere Wasserdampf, mit einem inerten Gas, insbesondere mit Stickstoff, $CO_2$, Helium, Neon, Argon sowie Mischungen davon.

Der im erfindungsgemäßen Verfahren eingesetzte rohe Ester entstammt einem üblichen Veresterungsverfahren. Derartige Verfahren sind dem Fachmann bekannt und in vielen Patentveröffentlichungen beschrieben. Dabei wird wenigstens eine Carbonsäure und/oder Carbonsäureanhydrid mit einem Alkohol oder Alkoholgemisch umgesetzt. Vielfach dient der Alkohol gleichzeitig als Schleppmittel für das bei der Umsetzung entstehende Reaktionswasser und wird daher im Überschuss eingesetzt. Vorzugsweise entfernt man vor dem Schritt a) aus dem rohen Ester die Hauptmenge des hier noch enthaltenen nicht umgesetzten Alkohols. Der Alkoholgehalt des im Schritt a) eingesetzten rohen Esters beträgt im Allgemeinen weniger als 5 Gew.-%, z. B. 1 bis 3 Gew.-%.

Im Veresterungsverfahren werden als Säurekomponente Carbonsäuren und/oder Carbonsäureanhydride eingesetzt. Bei mehrbasigen Carbonsäuren können auch teilweise anhydridisierte Verbindungen eingesetzt werden. Ebenso ist es möglich, Gemische aus Carbonsäuren und Anhydriden zu verwenden. Die Säuren können aliphatisch, einschließlich carbocyclisch, heterocyclisch, gesättigt oder ungesättigt, sowie aromatisch, einschließlich heteroaromatisch, sein.

Zu den geeigneten Carbonsäuren zählen aliphatische Monocarbonsäuren mit wenigsten 5 Kohlenstoffatomen, insbesondere 5 bis 20 Kohlenstoffatomen, wie n-Pentansäure, 2-Methylbuttersäure, 3-Methylbuttersäure, 2-Methylpentansäure, 2-Ethylbuttersäure, n-Heptansäure, Isoheptansäuren, 2-Methylhexansäure, Cyclohexancarbonsäure, n-Octansäure, 2-Ethylhexansäure, Isooctansäuren, n-Nonansäure, 2-Methyloctansäure, Isononansäuren, n-Decansäure, Isodecansäuren, 2-Methylundecansäure, Isoundecansäure, Tricyclodecancarbonsäure und Isotridecansäure.

Weiter eignen sich aliphatische $C_4$-$C_{10}$-Dicarbonsäuren bzw. deren Anhydride, wie Maleinsäure, Fumarsäure, Maleinsäureanhydrid, Bernsteinsäure, Bernsteinsäureanhydrid, Adipinsäure, Korksäure, Trimethyladipinsäure, Azelainsäure, Decandisäure, Dodecandisäure, Brassylsäure. Beispiele für carbocyclische Verbindungen sind: Hexahydrophthalsäureanhydrid (Cyclohexan-1,2-dicarbonsäureanhydrid), Hexahydrophthalsäure (Cyclohexan-1,2-dicarbonsäure), Cyclohexan-1,4-dicarbonsäure, Cyclohex-4-en-1,2-dicarbonsäure, Cyclohexen-1,2-dicarbonsäureanhydrid, 4-Methylcyclohexan-1,2-dicarbonsäure, 4-Methylcyclohexan-1,2-dicarbonsäureanhydrid, 4-Methylcyclohex-4-en-1,2-dicarbonsäure, 4-Methylcyclohex-4-en-1,2-dicarbonsäureanhydrid.

Beispiele geeigneter aromatischer Dicarbonsäuren bzw. deren Anhydride sind: Phthalsäure, Phthalsäureanhydrid, Isophthalsäure, Therephthalsäure, oder Naphthalindicarbonsäuren und deren Anhydride.

Beispiele geeigneter aromatischer Tricarbonsäuren bzw. deren Anhydride sind Trimellitsäure, Trimellisäureanhydrid oder Trimesinsäure; Beispiele einer geeigneten aromatischen Tetracarbonsäure bzw. ihres Anhydrids sind Pyromellitsäure und Pyromellitsäureanhydrid.

Besonders bevorzugt wird Phthalsäureanhydrid oder Adipinsäure als Carbonsäurekomponente eingesetzt.

Es werden bevorzugt verzweigte oder lineare aliphatische Alkohole mit 4 bis 13 C-Atomen eingesetzt. Die Alkohole sind einwertig und können sekundär oder primär sein.

Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im Veresterungsverfahren eingesetzt werden, sind beispielsweise n-Butanol, Isobutanol, n-Octanol(1), n-Octanol(2), 2-Ethylhexanol, Nonanole, Decylalkohole oder Tridecanole hergestellt durch Hydroformylierung oder Aldolkondensation und anschließende Hydrierung. Die Alkohole können als reine Verbindung, als Gemisch isomerer Verbindungen oder als Gemisch von Verbindungen mit unterschiedlicher C-Zahl eingesetzt werden. Z. B. können $C_9$/$C_{11}$-Alkoholgemische eingesetzt werden.

Bevorzugte Einsatzalkohole sind Gemische isomerer Octanole, Nonanole oder Tridecanole, wobei die letzteren aus den entsprechenden Butenoligomeren, insbesondere Oligomeren von linearen Butenen, durch Hydroformylierung und anschließender Hydrierung gewonnen werden können. Die Herstellung der Butenoligomeren kann im Prinzip nach drei Verfahren durchgeführt werden. Die sauer katalysierte Oligomerisierung, bei der technisch z. B. Zeolithe oder Phosphorsäure auf Trägern eingesetzt werden, liefert die verzweigtesten Oligomere. Bei Einsatz von linearen Butenen entsteht beispielsweise eine $C_8$-Fraktion, die im Wesentlichen aus Dimethylhexenen besteht (WO 92/13818). Ein ebenfalls weltweit ausgeübtes Verfahren ist die Oligomerisierung mit löslichen Ni-Komplexen, bekannt als DIMERSOL-Verfahren (B. Cornils, W. A. Herrmann, Applied Homogenous Catalysis with Organometallic Compounds, Seite 261-263, Verlag Chemie 1996). Weiterhin wird die Oligomerisierung an Nickel-FestbettKatalysatoren ausgeübt, wie beispielsweise der OCTOL-Process (Hydrocarbon Process., Int. Ed. (1986) 65 (2. Sect. 1), Seite 31-33).

Ganz besonders bevorzugte Einsatzstoffe für die erfindungsgemäße Veresterung sind Gemische isomerer Nonanole oder Gemische isomerer Tridecanole, die durch Oligomerisierung linearer Butene zu $C_8$-Olefinen und $C_{12}$-Ole-

finen nach dem Octol-Prozess, mit anschließender Hydroformylierung und Hydrierung hergestellt werden.

Weiterhin eignen sich Alkylenglykolmonoether, insbesondere Ethylenglykolmonoether, z. B. Ethylenglykolmono-$C_1$-$C1_8$-alkylether, wie Ethylenglykolmonomethylether, Ethylenglykolmonoethylether, Ethylenglykolpropylether, Ethylenglykolmonobutylether (2-Butoxy-ethanol) und deren Gemische; und Polyalkylenglykolmonoether insbesondere Polyethylenglykolmonoether, wie Polyethylenglykolmonomethylether.

Besonders bevorzugte Alkohohole sind 2-Ethylhexanol, 2-Propylheptanol, Isononanol-Isomerengemische, Decanol-Isomerengemische und $C_9$/$C_{11}$-Alkoholgemische sowie Ethylenglykolmonobutylether.

Geeigneter weise ist der Veresterungskatalysator unter Alkoholaten, Carboxylaten und Chelatverbindungen von Titan, Zirkonium, Zinn, Aluminium und Zink ausgewählt. Es eignen sich Tetraalkyltitanate, wie Tetramethyltitanat, Tetraethyltitanat, Tetra-n-propyltitanat, Tetra-isopropyltitanat, Tetra-n-butyltitanat, Tetra-isobutyltitanat, Tetrasec-butyltitanat, Tetraoctyltitanat, Tetra-(2-ethylhexyl)-titanat; Dialkyltitanate $((RO)_2TiO_2$, worin R z.B. für iso-Propyl, n-Butyl, iso-Butyl steht), wie Isopropyl-n-butyltitanat; Titan-Acetylacetonat-Chelate, wie Di-isopropoxy-bis(acetylacetonat)titanat, Di-isopropoxy-bis(ethylacetylacetonat)titanat, Di-n-butyl-bis(acetylacetonat)titanat, Di-n-butyl-bis(ethylacetoacetat)titanat, Tri-isopropoxid-bis(acetylacetonat)titanat; Zirkontetraalkylate, wie Zirkontetraethylat, Zirkontetrabutylat, Zirkontetrabutyrat, Zirkontetrapropylat, Zirkoncarboxylate, wie Zirkondiacetat; Zirkon-Acetylacetonat-Chelate, wie Zirkontetra(acetylacetonat), Tributoxyzirkonacetylacetonat, Dibutoxyzirkon(bis-acetylacetonat); Aluminiumtrisalkylate, wie Aluminiumtriisopropylat, Aluminiumtrisbutylat; Aluminium-Acetylacetonat-Chelate, wie Aluminiumtris(acetylacetonat) und Aluminiumtris(ethylacetylacetonat). Insbesondere werden Isopropyl-n-butyltitanat, Tetra(isopropyl)orthotitanat oder Tetra(butyl)orthotitanat eingesetzt.

Die Katalysatorkonzentration beträgt im allgemeinen 0,005 bis 1,0 Gew.-% bezogen auf das Reaktionsgemisch, insbesondere 0,01 bis 0,3 Gew.-%.

Der umzusetzende Alkohol, der als Schleppmittel dient, kann im stöchiometrischen Überschuss, bevorzugt 30 bis 200 %, besonders bevorzugt 50 bis 100 % der stöchiometrisch notwendigen Menge eingesetzt werden.

Das erfindungsgemäße Verfahren weist die folgenden Vorteile auf:

- Das erfindungsgemäße Verfahren gewährleistet stabile Agglomerationsbedingungen, die einen störungsfreien Verfahrensablauf ermöglichen.
- Das erfindungsgemäße Verfahren ermöglicht die Ausbildung gut filtrierbarer Agglomerate und verhindert ein sofortiges Zusetzen von Filtergeweben.
- Der Druckverlust über den Filterkuchen steigt langsamer an, wodurch Filtrationszykluslängen verlängert werden können und die Filter höher beladen werden können.
- Durch Vorlage des Wasser-untersättigten Rohesters im Agglomerier-Kessel wird eine schnelle und stabile Überführung der Veresterungskatalysator-Hydrolyseprodukte, neutralisierten Monoester und überschüssige Base enthaltenden Wassertropfen in die Agglomerate erreicht und eine Koagulation der Emulsion verhindert;
- Durch Vorgabe eines spezifischen Leistungseintrages im Emulgier-Kessel wird eine effektivere Überführung der suspendierten partikulären Veresterungskatalysator-Hydrolyseprodukte in die wässrige Phase erreicht und es kann die Wassertropfengröße und somit auf die Größe der Agglomerate Einfluss genommen und diese angepasst/gesteuert werden.

Die so hergestellten Ester aus mehrbasischen Carbonsäuren, wie beispielsweise Phthalsäure, Adipinsäure, Sebacinsäure, Maleinsäure, und aus Alkoholen, finden weite Anwendung in Lackharzen, als Bestandteile von Anstrichmitteln und insbesondere als Weichmacher für Kunststoffe. Spezielle Ester, die nach dem erfindungsgemäßen Verfahren aufgearbeitet werden können, sind Weichmacher für PVC, wie Dioctylphthalate, Diisononylphthalate, Diisodecylphthalate und Dipropylheptylphthalate; Weichmacher, beispielsweise für den Einsatz in Polyvinylbutyral, wie Dibutylglycoladipat, Dioctylazelat, Dioctyladipat, Dibutylsebacat, Di-(2-ethylhexyl)sebacat und Dioctylsebacat, sowie Dibutylglycolphthalat.

FIGUREN UND BEISPIELE

Die Erfindung wird durch die beigefügten Zeichnungen 1 und 2 sowie die folgenden Beispiele 1 bis 4 näher erläutert.

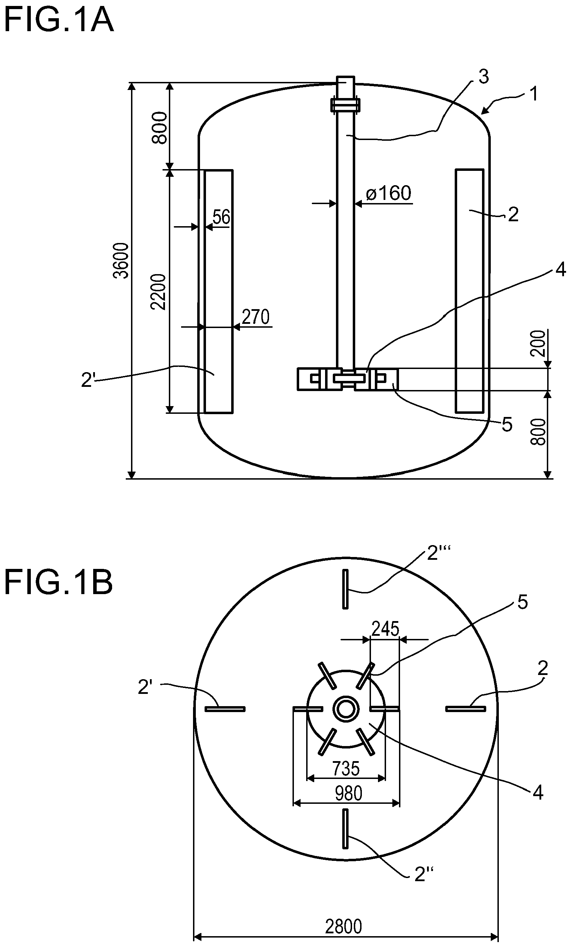

Fig. 1A: zeigt schematisch einen Längsschnitt durch eine beispielhafte Ausführungsform eines Emulgier-Kessel zur Durchführung von Schritt a) des erfindungsgemäßen Verfahrens,

Fig. 1B: zeigt schematisch einen Querschnitt durch eine beispielhafte Ausführungsform eines Emulgier-Kessel zur Durchführung von Schritt a) des erfindungsgemäßen Verfahrens,

Fig.: 2: zeigt beispielhaft den Zusammenhang zwischen Temperatur und Wassergehalt des Rohesters sowie die Löslichkeit von Wasser im Rohester.

In den Figuren 1A und 1B werden folgende Bezugszeichen verwendet:

1    Emulgier-Kessel
2    erster Strombrecher
2'    zweiter Strom brecher
2"    dritter Strom brecher
2'"    vierter Strombrecher
3    Rührwerk
4    Rührscheibe
5    Rührblatt

Fig. 1 stellt beispielhaft den Aufbau eines Emulgier-Kessels 1 zur Durchführung des erfindungsgemäßen Verfahrens dar. Der Emulgier-Kessel besteht aus einem Behälter in welchem ein Rührwerk 3 angeordnet ist. Beispielhaft ist das Rührwerk 3 als ein Scheibenrührer mit einer Rührscheibe 4 an welcher sechs Rührblätter 5 angeordnet sind, ausgestaltet. An der Behälterwandung sind vier Strombrecher 2, 2', 2", 2'" angeordnet. Als Antrieb für das Rührwerk 3 kann ein Motor mit 75 kW eingesetzt werden, um eine spezifische Leistung an der Rührerwelle im Bereich von 1 bis 3,5 $kW/m^2$ zu erreichen. Die Rührerdrehzahl kann insbesondere im Bereich zwischen 80 bis 140 U/min eingestellt werden. Die angeführten Bemaßungen sind Beispiele für den erfindungsgemäß beanspruchten Schlankheitsgrad H/D und die an der Behälterwandung angeordneten Strombrecher.

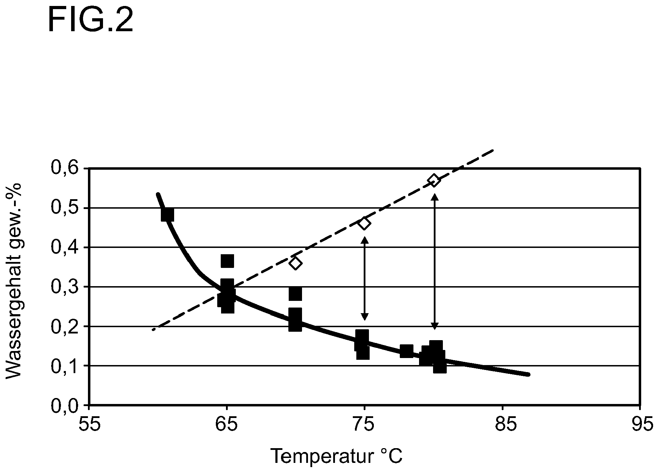

Die Figur 2 wird im nachfolgenden Beispiel 3 ausführlich beschrieben.

Beispiel 1

Ein Strom von 20.000 kg/h rohes Diisononylphthalat (DINP) mit einer Säurezahl von ca. 0,05 mg KOH/g und einem Alkoholgehalt von 2,0 Gew.-% wurde kontinuierlich aufgearbeitet.

Der DINP-Strom mit einer Temperatur von etwa 150 °C wurde unter einem Druck von 6 bar mit 100 kg/h 2 %iger wässriger Natriumhydroxidlösung (entsprechend ca. 200 % Überschuss, bezogen auf die Säurezahl des Rohesters) versetzt. Der gemischte Strom passierte eine Mischstrecke. Dann wurde der Strom in einen Rührbehälter auf etwa 100 mbar entspannt. Die Verweilzeit im Rührbehälter betrug etwa 0,5 h, während der das Gemisch mit einem dreistufigen Kreuzbalkenrührer bei 160 °C gerührt wurde.

Das Gemisch wurde in einen Emulgier-Kessel, wie in der Fig. 1 dargestellt, umgepumpt und dabei auf etwa 80 °C gekühlt. Im Emulgier-Kessel herrschte Umgebungsdruck. Der eingesetzte Rührer war ein Scheibenrührer mit 4 Stromstörern mit einer spezifischen Leistung an der Welle im Bereich von 1 bis 3,5 $kW/m^3$ bei der Drehzahl von 80 bis 140 U/min. Man setzte 800 kg/h Wasser (entsprechend 4 Gew.-%, bezogen auf den Rohesterstrom) zu. Die Verweilzeit im Emulgier-Kessel betrug etwa 0,5 h, während der das Gemisch mit einem Scheibenrührer (spezifischer Leistungseintrag: 3 W/l) intensiv durchmischt wurde bei 85 U/min.

Die Suspo-Emulsion wurde mit einer Temperatur von 80 °C in einen Agglomerier-Kessel übergeführt, in dem sich vorgelegtes, trockenes Diisononylphthalat aus einer vorhergehenden Produktion befand. Im Agglomerier-Kessel herrschte ein Druck von etwa 150 mbar. Die Verweilzeit im Agglomerier-Kessel betrug etwa 0,5 h, während der das Gemisch mit einem dreistufigen Kreuzbalkenrührer mit geringer Rührleistung von weniger als 0,1 W/l gerührt wurde. Die Wasser und Alkohol enthaltenden Brüden wurden abgezogen. Es stellte sich ein stationärer Wassergehalt von 0,12 Gew.-%. ein. Dieser Wert ist unterhalb der Löslichkeitsgrenze bei der gegebenen Temperatur und dem gegebenen Alkoholgehalt.

Die Suspo-Emulsion wurde in einen weiteren Agglomerier-Kessel mit einer Temperatur von 90 °C übergeführt. Im weiteren Rührbehälter herrschte ein Druck von etwa 100 mbar. Die Verweilzeit im weiteren Rührbehälter betrug etwa 0,5 h, während der das Gemisch mit einem dreistufigen Kreuzbalkenrührer mit geringer Rührleistung von weniger als 0,1 W/l gerührt wurde. Die Wasser und Alkohol enthaltenden Brüden wurden abgezogen.

Das Produkt wurde kontinuierlich über einen Zwischenbehälter einem Filter zugeführt und dort über ein Teflongewebe mit 10 μm Porenweite filtriert.

Man erhielt ein klares, von Katalysatorresten vollkommen freies Produkt mit einer Säurezahl von 0,01 mg KOH/g, einem Alkoholgehalt von 1,3 Gew.-% und einem Wassergehalt von 0,1 Gew.-%. Durch Strippen mit Wasserdampf konnte der Alkoholgehalt auf weniger als 0,01 Gew.-% vermindert werden.

Beispiel 2

Variation der Rührerdrehzahl im Emulgier-Kessel.

Das Beispiel 1 wurde wiederholt, wobei der volumenspezifische Leistungseintrag und die Umfangsgeschwindigkeit des Rühres im Emulfgier-Kessel variiert wurden, indem die Umdrehungszahl des Rührers im Emulgier-Kessel zwischen 55 bis 120 U/min stufenweise verändert wurde, wie in der nachfolgenden Tabelle 1 dargestellt.

Tabelle 1: Emulgier-Kesselparameter und Agglomerierverhalten

| Umdrehungen [U/min] | Umfangsgeschwindigkeit [m/s] | Volumenspezifischer Leistungseintrag [$kW/m^3$] | Beobachtung* |
|---|---|---|---|
| 114 | 5,8 | 2,18 | (+) |
| 120 | 6,2 | 2,54 | (-) Probe trüb |
| 114 | 5,8 | 2,18 | (+) Probe leicht trüb |
| 110 | 5,6 | 1,96 | (+) |
| 100 | 5,1 | 1,47 | (+) |
| 85 | 4,4 | 0,90 | (+) |
| 75 | 3,8 | 0,62 | (+) |
| 65 | 3,3 | 0,40 | (+) Pobe trüb |
| 55 | 2,8 | 0,24 | (+) Probe trüb |
| *(+): Agglomeratbildung (-): es erfolgte keine Agglomeratbildung | | | |

Die Erhöhung der Umdrehungszahl auf 120 U/min führte dazu, dass keine Agglomerate mehr gebildet wurden. Das erhaltene Produkt weist gegenüber dem Beispiel 1 einen höheren Filterwiderstand auf. Dadurch wird der Filter in kürzester Zeit verstopft und die Aufarbeitung muss angehalten werden. Die Erniedrigung der Umdrehungszahl auf unter 70 U/min führte zu einem trüben Ester; die Trübung weist auf die Anwesenheit von nicht agglomeriertem Veresterungskatalysator-Hydrolyseprodukt hin. Daneben waren Agglomerate sichtbar und das Produkt war filtrierbar.

Beispiel 3

Variation der Temperatur im Agglomerier-Kessel

Das Beispiel 1 wurde wiederholt, wobei die Temperatur im Agglomerier-Kessel im Bereich zwischen 60 bis 80°C in 5K Stufen variiert wurde. Aufgrund der Temperaturvariation änderte sich auch der Wassergehalt, da bei einem gleichbleibenden Druck von etwa 150 mbar bei niedrigerer Temperatur weniger Wasserdampf abgezogen wird. Bei jeder der Temperaturstufen wurden aus dem Kessel Proben gezogen und auf Wasser und Alkoholgehalt analysiert. Der Alkoholgehalt lag für alle Proben zwischen etwa 1,6 und 2 Gew.-%. Der Wassergehalt ist in der Figur 2 als durchgezogene Kurvenlinie dargestellt. In der Figur 2 ist außerdem die Löslichkeitskurve von Wasser im Rohester (bei einem angenommenen Alkoholgehalt von 2 Gew.-%, dargestellt als gestrichelte Trendgerade durch die unausgefüllten Rautepunkte) eingezeichnet. Oberhalb der Löslichkeitskurve liegt Wasser als diskrete Phase vor. Bis zu einer Temperatur von 65 °C und einem Wassergehalt von 0,27 Gew.-% konnte Produkt kontinuierlich filtriert werden und die gebildeten Agglomerate waren sichtbar. Wird allerdings die Probe mit den Agglomeraten auf Raumtemperatur abgekühlt, fällt das freie Wasser aus der Lösung aus und die Agglomerate werden innerhalb von Sekunden zersetzt, wobei sich ein weißer, wässriger Niederschlag auf dem Boden der Probenflasche ausbildete.

Vergleichsbeispiel

Die Erniedrigung der Temperatur im Agglomerier-Kessel im Verfahren von Beispiel 3 auf unter 65 °C bei einem Wassergehalt von über 0,5 Gew.-% resultierte in einem Ausfall des Agglomerationsprozesses. Das Produkt weist gegenüber dem Beispiel 1 einen höheren Filterwiderstand auf. Dadurch wird der Filter in kürzester Zeit verstopft und die Aufarbeitung muss angehalten werden. Wie aus Figur 2 ersichtlich, ist bei einer Temperatur unter 65 °C und einem Wassergehalt von 0,5 Gew.-% die Löslichkeitsgrenze von Wasser im Rohester überschritten und Wasser liegt als diskrete Phase vor. Unter diesen Bedingungen bilden sich keine stabilen Agglomerate aus.

Beispiel 4

Das Beispiel 1 wurde wiederholt, wobei die Säurezahl SZ des rohen Diisononylphthalat (DINP) im Bereich zwischen 0,045 bis 0,1 mg KOH pro g Lösung im Agglomerier-Kessel variiert wurde. Dabei wurde die Menge an Natronlauge so angepasst, dass der Rohester vollständig neutralisiert wurde (200% Überschuss). Bis zu einer SZ von 0,09 konnte Produkt kontinuierlich filtriert werden. Bei SZ von 0,1 weist das Produkt gegenüber dem Beispiel 1 einen höheren Filterwiderstand auf. Dadurch wird der Filter in kürzester Zeit verstopft und die Aufarbeitung muss angehalten werden.

**Patentansprüche**

1. Verfahren zur Aufarbeitung eines Rohesters, der Veresterungskatalysator-Hydrolyseprodukt in suspendierter partikulärer Form enthält, bei dem man

   a) den Rohester in einem Emulgier-Kessel mit 1 bis 10 Gew.-% Wasser versetzt und das Wasser unter Erhalt einer Suspo-Emulsion im Rohester emulgiert,
   b) die Suspo-Emulsion aus dem Emulgier-Kessel in einen Agglomerier-Kessel mit vorgelegtem, Wasser-untersättigten Rohester überführt, so dass sich im Gemisch der Suspo-Emulsion und des vorgelegten Rohesters ein Wassergehalt unterhalb der Löslichkeitsgrenze von Wasser im Rohester einstellt, wodurch die suspendierten partikulären Veresterungskatalysator-Hydrolyseprodukte stabile Agglomerate ausbilden, und
   c) die gebildeten Agglomerate abfiltriert.

2. Verfahren nach Anspruch 1, wobei der Rohester eine Säurezahl von kleiner als 0,1 mg KOH/g Rohester aufweist.

3. Verfahren nach einem der vorherigen Ansprüche, wobei man in Schritt a) das zugegebene Wasser mit mindestens einem Mischer bei einer Reynoldszahl von größer als $10^4$ bei einem volumenspezifischen Leistungseintrag im Bereich von 0,4 bis 2,54 kW/m$^3$ im Rohester emulgiert.

4. Verfahren nach einem der vorherigen Ansprüche, wobei man in Schritt a) das zugegebene Wasser mit mindestens einem Rührer bei einer Umfangsgeschwindigkeit im Bereich von 2,8 bis 6,2 m/s, vorzugsweise im Bereich von 3,3 bis 5,6 m/s, besonders bevorzugt im Bereich von 3,8 bis 5,1 m/s im Rohester emulgiert.

5. Verfahren nach einem der vorherigen Ansprüche, wobei die im Schritt a) erhaltene Suspo-Emulsion verteilte Wassertröpfchen mit einer mittleren Teilchengröße von mehr als 10 bis weniger als 2000 $\mu$m enthält.

6. Verfahren nach einem der vorherigen Ansprüche, wobei der Emulgier-Kessel einen Schlankheitsgrad H/D in einem Bereich von 1 bis 6, vorzugsweise im Bereich von 2 bis 4 aufweist.

7. Verfahren nach einem der vorherigen Ansprüche, wobei man in Schritt b) die Wasser-Unsättigung des im Agglomerier-Kessels vorgelegten Rohesters aufrecht erhält, indem man kontinuierlich Wasser abdestilliert.

8. Verfahren nach einem der vorherigen Ansprüche, wobei man im Agglomerier-Kessel die Suspo-Emulsion mit mindestens einem Mischer bei einem volumenspezifischen Leistungseintrag von kleiner als 0,2 kW/m$^3$ einmischt.

9. Verfahren nach einem der vorherigen Ansprüche, wobei man das Filtrat aus Schritt c) einer Strippbehandlung unterzieht.

10. Verfahren nach einem der vorhergehenden Ansprüche, wobei der suspendierte partikuläre Veresterungskatalysator-Hydrolyseprodukte enthaltende Rohester erhalten ist, indem man das Veresterungsprodukt einer Veresterungsreaktion, die mit einem metallhaltigen Veresterungskatalysator katalysiert ist, mit einer wässrigen Base versetzt und anschließend Wasser aus dem Veresterungsprodukt-Base-Gemisch abdampft.

11. Verfahren nach dem vorherigen Anspruch 10, wobei man dem Veresterungsprodukt die wässrige Base bei einer Temperatur T von mehr als 100 °C unter einem Druck p zusetzt, der gleich oder größer ist als der Dampfdruck von Wasser bei der Temperatur T.

12. Verfahren nach Anspruch 10 oder 11, wobei man Wasser aus dem Veresterungsprodukt-Base-Gemisch abdampft, indem man das Veresterungsprodukt-Base-Gemisch entspannt.

**Claims**

1. A process for workup of a crude ester comprising esterification catalyst hydrolysis product in suspended particulate form, in which

   a) the crude ester is admixed in an emulsifying tank with 1% to 10% by weight of water and the water is emulsified in the crude ester to obtain a suspoemulsion,
   b) the suspoemulsion from the emulsifying tank is transferred to an agglomerating tank containing an initial charge of water-undersaturated crude ester, such that a water content below the solubility limit of water in the crude ester is established in the mixture of the suspoemulsion and the initial charge of crude ester, as a result of which the suspended particulate esterification catalyst hydrolysis products form stable agglomerates, and
   c) the agglomerates formed are filtered off.

2. The process according to claim 1, wherein the crude ester has an acid number of less than 0.1 mg KOH/g crude ester.

3. The process according to any of the preceding claims, wherein the water added is emulsified in the crude ester in step a) with at least one mixer at a Reynolds number of greater than $10^4$ with a volume-specific power input in the range from 0.4 to 2.54 kW/m$^3$.

4. The process according to any of the preceding claims, wherein the water added is emulsified in the crude ester in step a) with at least one stirrer at a circumferential speed in the range from 2.8 to 6.2 m/s, preferably in the range from 3.3 to 5.6 m/s, more preferably in the range from 3.8 to 5.1 m/s.

5. The process according to any of the preceding claims, wherein the suspoemulsion obtained in step a) comprises distributed water droplets having a mean particle size of more than 10 to less than 2000 $\mu$m.

6. The process according to any of the preceding claims, wherein the emulsifying tank has a height/diameter ratio H/D within a range from 1 to 6, preferably in the range from 2 to 4.

7. The process according to any of the preceding claims, wherein the state of water unsaturation of the initial charge of crude ester in the agglomerating tank is maintained in step b) by distilling off water continuously.

8. The process according to any of the preceding claims, wherein the suspoemulsion is mixed in in the agglomerating tank with at least one mixer at a volume-specific power input of less than 0.2 kW/m$^3$.

9. The process according to any of the preceding claims, wherein the filtrate from step c) is subjected to a stripping treatment.

10. The process according to any of the preceding claims, wherein the crude ester comprising suspended particulate esterification catalyst hydrolysis products is obtained by admixing the esterification product of an esterification reaction catalyzed by a metallic esterification catalyst with an aqueous base and then evaporating water out of the esterification product/base mixture.

11. The process according to the preceding claim 10, wherein the aqueous base is added to the esterification product at a temperature T of more than 100°C under a pressure p equal to or greater than the vapor pressure of water at the temperature T.

12. The process according to claim 10 or 11, wherein water is evaporated out of the esterification product/base mixture by decompressing the esterification product/base mixture.

**Revendications**

1. Procédé pour le traitement d'un ester brut, qui contient un produit d'hydrolyse de catalyseur d'estérification sous forme particulaire en suspension, dans lequel on

   a) met en réaction l'ester brut dans une cuve d'émulsification avec 1 à 10 % en poids d'eau et on émulsifie l'eau dans l'ester brut avec obtention d'une suspoémulsion,

b) on transfère la suspoémulsion de la cuve d'émulsification dans une cuve d'agglomération comportant de l'ester brut sous-saturé en eau prédéposé, de sorte que, dans le mélange de la suspoémulsion et de l'ester brut prédéposé, s'ajuste une teneur en eau en dessous de la limite de solubilité de l'eau dans l'ester brut, les produits d'hydrolyse particulaires en suspension du catalyseur d'estérification formant ainsi des agglomérats stables, et

c) on filtre les agglomérats formés.

2. Procédé selon la revendication 1, l'ester brut présentant un indice d'acide inférieur à 0,1 mg de KOH/g d'ester brut.

3. Procédé selon l'une quelconque des revendications précédentes, où dans l'étape a) on émulsifie l'eau ajoutée dans l'ester brut avec au moins un mélangeur avec un nombre de Reynolds supérieur à $10^4$ à une puissance d'entrée spécifique en volume dans la plage de 0,4 à 2,54 kW/m$^3$.

4. Procédé selon l'une quelconque des revendications précédentes, où dans l'étape a) on émulsifie l'eau ajoutée dans l'ester brut avec au moins un agitateur à une vitesse périphérique dans la plage de 2,8 à 6,2 m/s, de préférence dans la plage de 3,3 à 5,6 m/s, particulièrement préférablement dans la plage de 3,8 à 5,1 m/s.

5. Procédé selon l'une quelconque des revendications précédentes, où la suspoémulsion obtenue dans l'étape a) contient des gouttelettes d'eau réparties dotées d'une grosseur moyenne de particule supérieure à 10 jusqu'à inférieure à 2 000 $\mu$m.

6. Procédé selon l'une quelconque des revendications précédentes, la cuve d'émulsification présentant un coefficient d'élancement H/D dans une plage de 1 à 6, de préférence dans la plage de 2 à 4.

7. Procédé selon l'une quelconque des revendications précédentes, où dans l'étape b) on maintient l'insaturation en eau de l'ester brut prédéposé dans la cuve d'agglomération en distillant de l'eau de manière continue.

8. Procédé selon l'une quelconque des revendications précédentes, où on mélange la suspoémulsion dans la cuve d'agglomération avec au moins un mélangeur à une puissance d'entrée spécifique en volume inférieure à 0,2 kW/m$^3$.

9. Procédé selon l'une quelconque des revendications précédentes, où on soumet le filtrat de l'étape c) à un traitement de strippage.

10. Procédé selon l'une quelconque des revendications précédentes, l'ester brut contenant des produits d'hydrolyse du catalyseur d'estérification particulaire en suspension étant obtenu en faisant réagir le produit d'estérification d'une réaction d'estérification, qui est catalysée par un catalyseur d'estérification contenant un métal, avec une base aqueuse et ensuite en distillant l'eau du mélange produit d'estérification-base.

11. Procédé selon la revendication précédente 10, où on ajoute la base aqueuse au produit d'estérification à une température T supérieure à 100 °C sous une pression p, qui est égale ou supérieure à la pression de vapeur de l'eau à la température T.

12. Procédé selon la revendication 10 ou 11, où on distille de l'eau du mélange produit d'estérification-base, en détendant le mélange produit d'estérification-base.

## FIG.1A

## FIG.1B

FIG.2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2010076193 A1
- DE 1945359
- DE 2330435
- EP 1300388 A
- US 5434294 A
- WO 9711048 A
- DE 19721347
- US 20060270868 A1
- WO 9213818 A

### In der Beschreibung aufgeführte Nicht-Patentliteratur

- **B. CORNILS ; W. A. HERRMANN.** Applied Homogenous Catalysis with Organometallic Compounds. Verlag Chemie, 1996, 261-263
- *Hydrocarbon Process., Int. Ed.,* 1986, vol. 65, 31-33